# EUROPEAN PATENT SPECIFICATION

(11) **EP 2 280 028 B1**
(45) Date of publication and mention of the grant of the patent: **27.03.2013**
(21) Application number: 09754702.0
(22) Date of filing: 26.05.2009
(51) Int. Cl.: C07K 14/47, G01N 33/68

(54) **METHOD FOR EFFICIENTLY AMPLIFYING ABNORMAL PRION PROTEIN DERIVED FROM SHEEP SCRAPIE**
VERFAHREN ZUR EFFIZIENTEN AMPLIFIZIERUNG VON AUS SCRAPIE ABGELEITETEN ABNORMALEN PRIONPROTEINEN
PROCÉDÉ D'AMPLIFICATION EFFICACE D'UNE PROTÉINE PRION ANORMALE DÉRIVÉE DE LA TREMBLANTE DU MOUTON

(30) Priority: 28.05.2008 JP 2008139280
(43) Date of publication of application: 02.02.2011
(73) Proprietor: Incorporated Administrative Agency National Agriculture and Food Research Organization, Ibaraki 305-8517 (JP)
(72) Inventor: MURAYAMA, Yuichi, Tsukuba-shi Ibaraki 305-0856 (JP); MASUJIN, Kentaro, Tsukuba-shi Ibaraki 305-0856 (JP); YOKOYAMA, Takashi, Tsukuba-shi Ibaraki 305-0856 (JP); MOHRI, Shirou, Tsukuba-shi Ibaraki 305-0856 (JP)
(74) Representative: Hiebl, Inge Elisabeth
(86) International application number: PCT/JP2009/059617
(87) International publication number: WO 2009/145194

(56) References cited:
- WO-A1-2004/113925
- WO-A1-2005/001481
- WO-A2-2006/113915
- JP-A- 2004 292 437
- US-A1- 2006 154 239
- HORIUCHI M ET AL: "INTERACTIONS BETWEEN HETEROLOGOUS FORMS OF PRION PROTEIN: BINDING, INHIBITION OF CONVERSION, AND SPECIES BARRIERS", PROCEEDINGS OF THE NATIONAL ACADEMY OF SCIENCES OF USA, NATIONAL ACADEMY OF SCIENCE, WASHINGTON, DC; US, vol. 97, no. 11, 23 May 2000 (2000-05-23), pages 5836-5841, XP000973870, ISSN: 0027-8424, DOI: 10.1073/PNAS.110523897
- LI ET AL: "Species barriers for chronic wasting disease by in vitro conversion of prion protein", BIOCHEMICAL AND BIOPHYSICAL RESEARCH COMMUNICATIONS, ACADEMIC PRESS INC. ORLANDO, FL, US, vol. 364, no. 4, 25 October 2007 (2007-10-25), pages 796-800, XP022344192, ISSN: 0006-291X, DOI: 10.1016/J.BBRC.2007.10.087
- SOTO C ET AL: "Pre-symptomatic detection of prions by cyclic amplification of protein misfolding", FEBS LETTERS, ELSEVIER, AMSTERDAM, NL, vol. 579, no. 3, 31 January 2005 (2005-01-31), pages 638-642, XP004725170, ISSN: 0014-5793, DOI: 10.1016/J.FEBSLET.2004.12.035
- MURAYAMA, Y. ET AL.: 'Efficient in vitro amplification of a mouse-adapted scrapie prion protein.' NEUROSCIENCE LETTERS vol. 413, 2007, pages 270 - 273, XP005880312

## Description

### TECHNICAL FIELD

The present invention relates to *in vitro* methods for efficiently amplifying abnormal prion protein derived from sheep scrapie.

### BACKGROUND ART

The first disease discovered as one of the diseases caused by propagation of abnormal prion protein is scrapie, which is a disease associated with ataxia found in sheep and is characterized by spongy vacuolation in the brain. Later, bovine spongiform encephalopathy (BSE) also referred to as mad cow disease, Creutzfeldt Jakob disease (CJD) in humans, Gerstmann-Straussler-Scheinker syndrome (GSS) or the like became known as the diseases caused by propagation of abnormal prion protein.

These diseases are not caused by viral infection and already-known pathogens have not been discovered. A specific protein is commonly found in these diseases, and thus is believed to be an etiologic agent that causes transmission and infection. A "proteinaceous infectious particle: prion" has been proposed and the specific diseases described above have been called prion diseases.

Incidentally, it has been found that the human prion protein gene is on chromosome 20 and a prion protein is composed of 235 amino acids. The main component of the infectious agent, prion, is believed to be this prion protein. The prion protein that the infectious agent is composed of is called a scrapie form or abnormal form of prion protein (PrP^{sc}), and a normal form of prion protein is called normal prion protein (PrP^{c}).
Since prion diseases have been found in many animal species including humans, and the accumulation of abnormal prion protein (PrP^{Sc}), which is generally resistant to proteases, is observed in infected individuals, this Prp^{Sc} is believed to be involved in the diseases as a main etiologic agent.

Transmission of prion diseases may occur between different animal species. For example, cows might have been infected with scrapie via animal feed containing contaminated materials derived from sheep infected with scrapie and consequently have developed BSE. Furthermore, it has been thought that cats that ate feed containing materials derived from cows infected with BSE developed feline spongiform encephalopathy (FSE). Accordingly, recent research has reported that BSE, in particular, has a high potential to infect humans (Non-Patent Document 1).

Scrapie, which is a prion disease found in sheep and goats, spread pandemically in Europe in the 18th century. Scrapie also has occurred in Japan since 1948 although it is sporadic.
Incidentally, such simple diagnostic methods as used for diagnosis of other infectious diseases or metabolic diseases cannot be applied to scrapie-infected sheep, that is, sheep infected with abnormal prion protein (PrP^{Sc}) and scrapie also has a long incubation period. Therefore, a practical antemortem diagnostic method for scrapie-infected sheep has not been established. Moreover, since only a trace amount of PrP^{Sc} is expected to exist in biological materials such as blood, a supersensitive detection technique with a detection limit far superior to that of conventional methods needs to be developed for an antemortem diagnosis.

On the other hand, abnormal prion protein (PrP^{Sc}) also has a feature in that it is not inactivated by ordinal sterilization. A bioassay is generally used to determine inactivation of PrP^{Sc}, and the bioassay includes inoculating a laboratory animal such as a mouse with PrP^{Sc} that is thought to have been inactivated and determining whether the mouse develops symptoms or not, thereby detecting the infectivity of PrP^{Sc}. However, this assay requires long-term breeding and observation of a laboratory animal and the result is only obtained after several tens to several hundreds of days, and therefore it is problematic in terms of the enormous amount of time and money required for follow-up.
Accordingly, if a highly sensitive method by which PrP^{Sc} remaining after inactivation can be detected in a short period of time is successfully developed, the development of a method for inactivating prion and the examination of prion inactivation will be substantially improved.

One of the conventional methods developed to detect abnormal prion protein (PrP^{Sc}) is a PMCA (protein misfolding cyclic amplification) method where PrP^{Sc} is amplified by mixing a brain homogenate infected with prion and a normal brain homogenate *in vitro* and repeating sonication and incubation with stirring. The PMCA method enabled detection of a trace amount of PrP^{Sc} (Non-Patent Document 2 and Patent Document 1).

PrP^{Sc} used in the PMCA method derived from a brain homogenate derived from a hamster infected with scrapie (hamster-adapted 263K strain). The PMCA method is a method for amplifying PrP^{Sc} by repeating a series of mixing-incubation-sonication cycles, wherein the method includes diluting the above mentioned brain homogenate, adding a brain homogenate derived from a normal hamster, which serves as normal prion protein (PrP^{c}), to the diluted brain homogenate and mixing them together, incubating the mixture *in vitro,* allowing conformational conversion of excessively added PrP^{c} into PrP^{Sc} and amplifying PrP^{Sc}, sonicating the resulting product to micronize aggregated PrP^{Sc}, and re-incubating the micronized PrP^{Sc} with excessive PrP^{c}.

The PMCA method is highly effective in the amplification of PrP^{Sc} from a hamster model of scrapie infection. The average amplification factor of PrP^{Sc} after repeating 5 cycles is 58, and repeating 10 cycles allow for detection of even PrP^{Sc} samples diluted 10,000 or more-fold. Thus, a trace amount of PrP^{Sc} can be detected in body fluids such as the blood (Non-Patent Document 3) and the urine (Non-Patent Document 4) obtained from an infected hamster, and also PrP^{sc} can be detected in blood cells derived from an animal during the incubation period (Non-Patent Documents 4 and 5). Thus, the PMCA method has been shown to be useful as an early diagnostic method of prion diseases and an examination method of prion inactivation.

US2006/0154239 discloses a PMCA method using bovine PrP^{Sc} and hamster PrP^{c}.
However, although the proposed PMCA method is highly effective in the amplification of PrP^{Sc} from a hamster model of scrapie infection and of bovine PrP^{sc} it does not provide sufficient amplification when applied to the amplification of the abnormal prion protein (PrP^{Sc}) from a scrapie-infected sheep. Accordingly, the PMCA method is not satisfactorily applicable to an antemortem diagnostic method or an early diagnostic method of sheep scrapie.
[Patent Document 1] Japanese Patent Application Laid-Open No. 2004-503748
[Non-Patent Document 1] Nature, 383: p685-690 (1996)
[Non-Patent Document 2] Nature, 411: p810-813 (2001)
[Non-Patent Document 3] Nat. Med., 11: p982-985 (2005)
[Non-Patent Document 4] J. Gen. Virol., 88: p2890-2898 (2007)
[Non-Patent Document 5] Science, 313: p92-94 (2006)
[Non-Patent Document 6] FEBS Lett., 579: p638-642 (2005)

### DISCLOSURE OF THE INVENTION

### PROBLEMS TO BE SOLVED BY THE INVENTION

In view of the above circumstances, it is a primary object of the present invention to provide a method for efficiently amplifying abnormal prion protein (PrP^{Sc}) derived from sheep scrapie. An ultimate object of the present invention is to eradicate the transmission of a prion disease by detecting a scrapie-infected sheep early and to develop a method for inactivating prions and permit early examination of prion inactivation.

### MEANS FOR SOLVING THE PROBLEMS

To solve the above problems, the present inventors enabled abnormal prion protein (PrP^{Sc}) derived from sheep scrapie to be amplified efficiently by modifying the conventional PMCA method and accomplished the present invention.

The PMCA method proposed until now, which enables amplification of PrP^{Sc} from a hamster model of scrapie infection, amplifies PrP^{Sc} by mixing the PrP^{Sc} to be amplified with PrP^{c} derived from a normal homologous animal (hamster).
A homologous animal is used because the amplification using a heterologous animal generally results in lower amplification efficiency or no amplification compared to the amplification using a homologous animal (Non-Patent Document 6).
The present inventors carried out examinations to improve the method in this respect. The present inventors found that, in the amplification of PrP^{Sc} derived from sheep scrapie, using normal prion protein (PrP^{c}) derived from a heterologous animal as a source (mouse) surprisingly leads to a substantial increase of the amplification efficiency of PrP^{Sc}, thereby enabling detection of a trace amount of PrP^{Sc} derived from sheep scrapie, which a conventional PMCA method had not successfully detected. Thus, the present inventors accomplished the present invention.

Accordingly, the present invention provides a method for efficiently amplifying abnormal prion protein (PrP^{Sc}) derived from sheep scrapie, wherein the method is based on a PMCA (protein misfolding cyclic amplification) method in which normal prion protein (PrP^{c}) is used as a source and PrP^{Sc} is used as a seed, and PrP^{Sc} derived from sheep scrapie is amplified by mixing, incubating, and sonicating both the PrP^{c} and the PrP^{Sc} repeatedly, and wherein PrP^{c} derived from a heterologous animal is used as the PrP^{c}, wherein the heterologous animal is mouse.
More specifically, the present invention provides a method for efficiently amplifying PrP^{Sc} derived from sheep scrapie as described above, wherein the PrP^{c} used as a source derives from a brain homogenate containing PrP^{c}, and the PrP^{Sc} derived from sheep scrapie which is used as a seed derives from a body tissue containing PrP^{Sc} derived from a sheep scrapie-infected animal.

### EFFECTS OF THE INVENTION

The method of the present invention enables a trace amount of PrP^{Sc} derived from sheep scrapie to be detected. The sensitivity in detecting PrP^{Sc} derived from sheep scrapie by using the method of the present invention is remarkably efficient compared to preexisting detection methods such as the ELISA method. Thus, the method of the present invention is advantageous in that sensitivity higher than that in a bioassay is obtained by repeating amplification reactions as appropriate.
Moreover, the method of the present invention can avoid spending an enormous amount of time and money, which a conventional bioassay requires for detection of PrP^{Sc}, and thus it also has excellent practicality and rapidity.

Accordingly, the method of the present invention permits an antemortem diagnosis and an early diagnosis of sheep scrapie. Moreover, the method permits rapid examination of inactivation of abnormal prion protein derived from sheep scrapie, thereby contributing to establishing a method for inactivating abnormal prion protein. This method also has the advantage that it is applicable to a safety evaluation method of raw materials for feed and fertilizer such as meat and bone meal, environmental monitoring of, for example, PrP^{Sc} in soil and the like.
In particular, the occurrence of sheep scrapie has been confirmed not only in Japan but also in most sheep-breeding countries with the exception of Australia and New Zealand, and the risk of occurring in the future should be taken into consideration in countries with no existing sheep scrapie. The method of the present invention is useful as a communicable disease control measure for sheep scrapie for organism (animal) import and also as a preventive measure against sheep scrapie for imported mutton.

### BRIEF DESCRIPTION OF THE DRAWINGS

Fig. 1 shows the results of amplification in which PrP^{c} sources from different heterologous animals were added in accordance with Test Example 1 of the present invention.
Fig. 2 shows the results of amplification in which PrP^{c} sources from different heterologous animals were added in accordance with Test Example 1 of the present invention.
Fig. 3 shows the results of examination of the concentrations of PrP^{Sc} added as a seed in accordance with Test Example 2 of the present invention.
Fig. 4 shows the results of examination of the concentrations of PrP^{Sc} added as a seed in accordance with Test Example 2 of the present invention.
Fig. 5 shows the results of examination of the concentrations of PrP^{Sc} added as a seed in accordance with Test Example 3 of the present invention.
Fig. 6 shows the results of examination of the amplification of PrP^{Sc} derived from genotypically different scrapie-infected sheep in accordance with Test Example 4 of the present invention.
Fig. 7 shows the results of examination of the amplification of PrP^{Sc} derived from genotypically different scrapie-infected sheep in accordance with Test Example 4 of the present invention.
Fig. 8 shows the results of detection of PrP^{Sc} in blood of scrapie-infected sheep in accordance with Test Example 5 of the present invention.

### BEST MODE FOR CARRYING OUT THE INVENTION

As described above, the basic embodiment of the present invention is an amplification method for efficiently amplifying abnormal prion protein (PrP^{Sc}) derived from sheep scrapie, wherein the method is based on a PMCA method in which normal prion protein (PrP^{c}) is used as a source and PrP^{Sc} is used as a seed, and PrP^{Sc} from sheep scrapie is amplified by stir-mixing, incubating, and sonicating both the PrP^{c} and the PrP^{Sc} repeatedly, and wherein PrP^{c} derived from the heterologous animal mouse is used as the PrP^{c}.

Since PrP^{Sc} from a scrapie-infected sheep is intended to be amplified, normal prion protein (PrP^{c}) used as a source in the present disclosure is PrP^{c} derived from a heterologous animal other than a sheep. Such PrP^{c}s derived from a heterologous animal may include various PrP^{c}s, and specifically, PrP^{c} selected from PrP^{c} derived from rodents such as a mouse or a rat.
Among others, especially using mouse PrP^{c} as a source can increase the amplification efficiency of PrP^{Sc}.
However, although goats, cows, pigs, hamsters, chickens, squirrel monkeys, and cynomolgus monkeys are heterologous animals, PrP^{Sc} was not amplified by using PrP^{c} derived therefrom as a source (see Test Examples described below).

Body tissue of the heterologous animals can be used to provide normal prion protein (PrP^{c}) which is used as a source. Preferably, a brain homogenate is used.
The brain homogenate is prepared by grinding (homogenating) the brain of the heterologous animals, for example in a mortar. More specifically, 10% (w/v) suspension that is prepared by homogenizing the brain of a normal heterologous animal in PBS with 1% Triton X-100 (t-octylphenoxy polyethoxyethanol) and 4 mM EDTA (containing a protease inhibitor) is preferably used.

On the other hand, it is preferable to use body tissue of a scrapie-infected sheep to provide PrP^{Sc} derived from sheep scrapie which is used as a seed. Any body tissue, for example, brain tissue, blood tissue, and urine derived from a scrapie-infected sheep can be used as such a body tissue.
Thus, the amplification method provided by the present invention enables even a trace amount of PrP^{Sc} to be amplified efficiently. Accordingly, the novel PMCA method enables detection of samples containing a trace amount of abnormal prion protein derived from sheep scrapie and increases the sensitivity in detecting a scrapie-infected sheep. Thus, PrP^{Sc} derived from sheep scrapie which is used as a seed is not particularly limited and any body tissue derived from a scrapie-infected sheep can be used.

Incidentally, when amplification is performed according to the conventional PMCA method for amplifying PrP^{Sc} from a hamster model of scrapie infection, amplification is usually achieved by adding a surfactant to abnormal prion protein and solubilizing PrP^{Sc}, and then mixing the solubilized PrP^{Sc} with PrP^{c} allowing conformational conversion of PrP^{c} used as a source into PrP^{Sc}.
The PMCA method of the present invention is also performed in the presence of a surfactant. Such surfactant is a non-ionic surfactant, and preferably t-octylphenoxy polyethoxyethanol (Triton X-100), polyoxyethylene (9) octylphenyl ether (NP-40) or the like is used without limitation.

The general operational procedures of the method of the present invention are described below.
That is, the method of the present invention uses normal prion protein (PrP^{c}) derived from a heterologous animal as a source and PrP^{Sc} derived from sheep scrapie as a seed, and stir-mixes and incubates both PrP^{c} and PrP^{Sc}, as described above. The incubating (incubation) condition is not necessarily limited and an optimal incubation condition may be selected as appropriate. Specifically, for example, the method may include incubation for about one hour at 37°C with stirring.

This stir-mixing and incubation allows the conformational conversion of Pr^{c} derived from a heterologous animal added as a source into PrP^{Sc} in part. The aggregates of conformationally converted PrP^{Sc} are dispersed by sonication and the cycle of stir-mixing and incubation with excessive Pr^{c} is repeated again.
The sonication is not particularly limited and the sonication used in the usual PMCA method may be used without any change. For example, sonication may be performed on Digital Sonifier 450D (Branson) or 070-GOT (ELECON Corporation).
Although sonication conditions may vary with the systems used and are not necessarily limited, an exemplary condition is, for example, about 5 cycles of a 0.2 second oscillation and a 0.1 second pause or a 3 second oscillation and a 1 second pause with output setting at 100%.

While the cycles of stir-mixing-incubation-sonication are repeated, PrP^{c} added as a source is conformationally converted to PrP^{Sc} sequentially, so that PrP^{Sc} is amplified.
To achieve one round of amplification by the method of the present invention, in general, the cycles described above are preferably performed 20 to 40 times per one round of amplification process.
Then, the number of cycles depends on the concentrations of PrP^{c} used as a source and of PrP^{Sc} used as a seed and is not limited.

After the amplification (repeating the cycles of incubation with stirring and sonication) of PrP^{Sc} is completed, the reactant obtained is subjected to a degradation process using proteases for degradation.
Since abnormal prion protein is generally resistant to proteases, it is necessary to degrade normal prion protein to recover this protein specifically. Thus, this degradation process is a process in which proteins other than the prion protein are degraded and additionally the normal prion protein is also degraded.
Proteases may include proteinase K. It is desirable to use proteinase K for degradation.

Incidentally, amplified PrP^{Sc} can be detected by Western blotting. Specifically, a sample is electrophoresed and separated on 15% SDS-PAGE and transferred to a membrane. After blocking, the membrane is reacted with HRP-labelled anti-T2 antibodies. Subsequently, after washing the membrane, amplified PrP^{Sc} can be detected and identified by detecting a luminous reaction using Immobilon Western.

The inventors' studies demonstrated that amplified PrP^{Sc} in even a 10⁻¹⁰-fold dilution prepared from a brain homogenate derived from a scrapie-infected sheep can be detected by repeating amplification reactions 5 times (one round of amplification consists of 40 cycles of incubation with stirring and sonication).
Thus, the method of the present invention has extremely good practicality and rapidity, has a great possibility of being used as a diagnosis, a safety evaluation method, and a preventive measure regarding sheep scrapie, and has an extensive applicability.

Incidentally, a sheep PrP gene is known to have mutations that cause various amino acid substitutions. Accordingly, selection and mating of improved sheep have been performed recently to obtain a solid with enhanced resistance to sheep scrapie.
The inventors' studies demonstrated that PrP^{Sc} also could be amplified efficiently by the method of the present invention, even if PrP^{Sc} derived from genotypically different sheep (see Test Examples described below).

### [EXAMPLES]

Herein below, the present invention will be described in more detail with reference to Test Examples instead of Implementation Examples, but these Test Examples are not meant to limit the present invention in any way.

### Test Example 1: Amplification of PrP^{Sc} Derived from Sheep Scrapie (Examination of Source)

### 1. Method

Abnormal prion protein (PrP^{Sc}) derived from sheep scrapie was amplified using a PMCA (protein misfolding cyclic amplification) method.
Ten percent brain homogenates derived from a normal sheep, which was a homologous animal, and a goat, a mouse, a squirrel monkey, a cow, a pig, a chicken, a hamster, a rat, and a cynomolgus monkey, which were heterologous animals, were used as normal prion protein (PrP^{c}) sources.
On the other hand, a 10⁻¹-fold dilution prepared from a 10% brain homogenate derived from a scrapie-infected sheep from the United Kingdom was used as a PrP^{Sc} seed.
After PMCA amplification, the samples were digested by proteinase K and the signal from protease-resistant PrP (PrP^{RES}) was detected by Western blotting.

### 2. Result

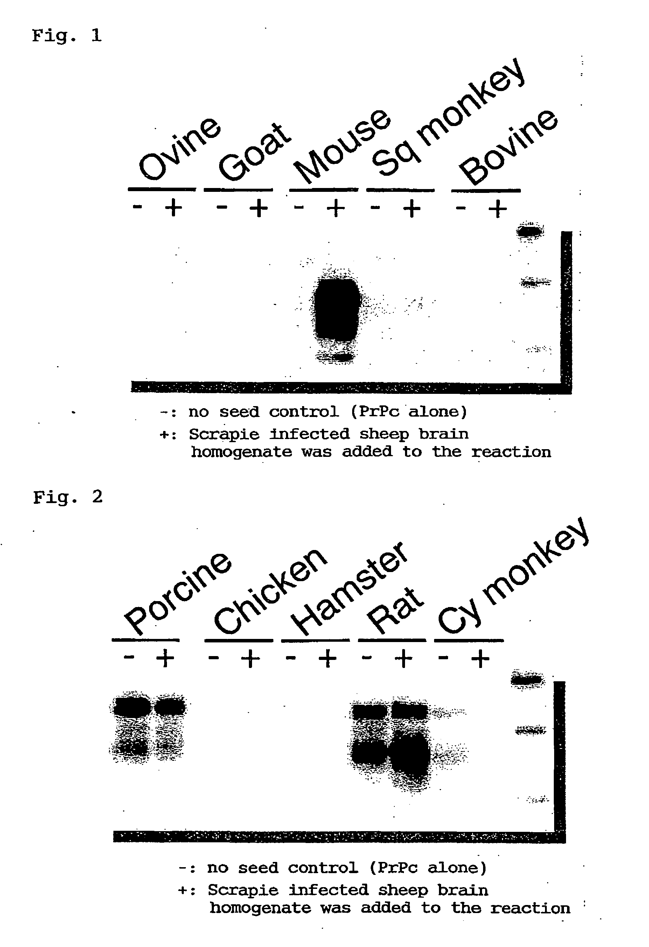

The results are shown in Figs. 1 and 2. Fig. 1 shows the results when a sheep, a goat, a mouse, a squirrel monkey, and a cow were used as a PrP^{c} source and Fig. 2 shows the results when a pig, a chicken, a hamster, a rat, and a cynomolgus monkey were used as a PrP^{c} source.
As is understood from these results, while little amplification of PrP^{Sc} was found when PrP^{c} derived from a homologous animal (sheep) was used as PrP^{c} used as a source, effective amplification of PrP^{Sc} was found when PrP^{c} derived from a mouse, which was a heterologous animal, was used.
Also, when PrP^{c} derived from a rat, which was a heterologous animal, was used, stronger signal was found compared to a control with no added seed, and amplification was confirmed although the efficiency was lower than that using a mouse. On the other hand, when PrP^{c} derived from a goat, a cow, a chicken, and a hamster was used as a source, no signal was observed. When PrP^{c} derived from a squirrel monkey, a pig, and a cynomolgus monkey was used, the signal intensity of a control with no added seed was stronger than that of samples amplified in the presence of an added brain homogenate derived from an infected sheep. There was no specific amplification of PrP^{Sc} when PrP^{c} from these heterologous animals was used.

### Test Example 2: Amplification of PrP^{Sc} Derived from Sheep Scrapie (Examination of Sensitivity 1)

The results from Test Example 1 indicated that the amplification of PrP^{Sc} was highly effective especially when mouse PrP^{c} was used as a source among heterologous animals. Thus, the present inventors examined how the concentration of PrP^{Sc} derived from sheep scrapie used as a seed and the number of amplification reactions affected amplification of PrP^{Sc}.

### I

### 1. Method

PrP^{Sc} derived from a scrapie-infected sheep was amplified using the PMCA method as with Test Example 1.
A brain homogenate derived from a scrapie-infected sheep was 10⁻¹ to 10⁻³-fold diluted and used as PrP^{Sc}.
PrP^{c} derived from an ICR mouse, which was a heterologous animal, was used as PrP^{c} as a source. PrP^{c} derived from a sheep, which was a homologous animal, served as a control for comparison.
Furthermore, a sample with no added seed served as a control with no additive.

### 2. Result

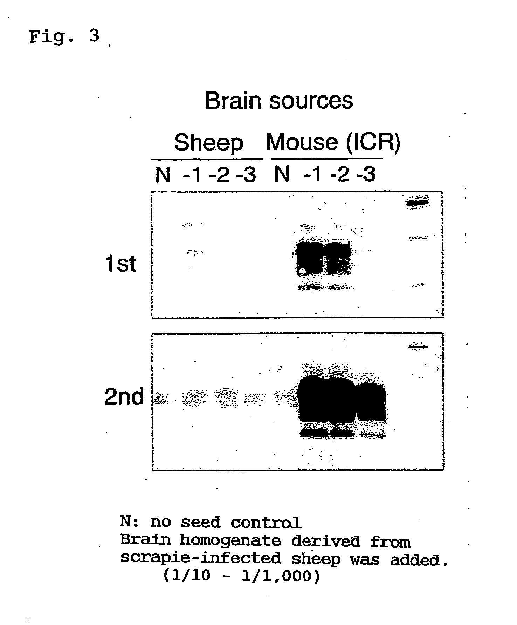

The results are shown in Fig. 3. As is understood from the results shown in Fig. 3, even if a brain homogenate derived from a scrapie-infected sheep was 10⁻³-fold diluted, effective amplification of PrP^{Sc} was achieved by performing 2 rounds of the amplification reaction, where one round of the amplification reaction consisted of 40 cycles.
In contrast, when PrP^{c} of a sheep, which was a homologous animal, was used as a source, little amplification was observed after two rounds of the amplification reaction.

### II

### 1. Method

PrP^{Sc} derived from a scrapie-infected sheep was amplified using the PMCA method as with section I above described.
A 10% brain homogenate derived from a scrapie-infected sheep was 10⁻¹ to 10⁻⁸-fold diluted and used as PrP^{Sc}.
PrP^{c} derived from an ICR mouse, which was a heterologous animal, was used as PrP^{c} as a source. A sample with no added seed served as a control with no additive.

### 2. Result

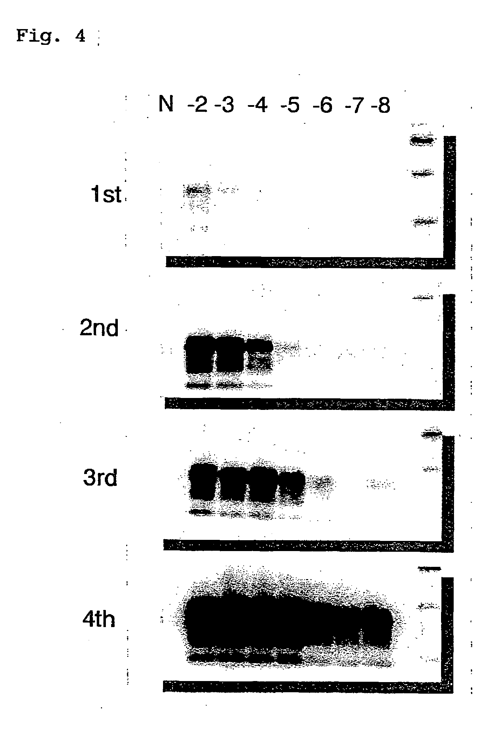

The results are shown in Fig. 4. As is understood from the results shown in Fig. 4, it was found that even if a brain homogenate derived from a scrapie-infected sheep was 10⁻⁸-fold diluted, good amplification of PrP^{Sc} was achieved by performing 4 rounds of the amplification reaction repeatedly. The results help understand the specificity of the present invention.

### Test Example 3: Amplification of PrP^{Sc} Derived from Sheep Scrapie (Examination of Sensitivity 2)

Test Example 2 described above demonstrated that when mouse PrP^{c} was used as a source and a brain homogenate derived from a scrapie-infected sheep was used as PrP^{Sc}, the efficient amplification of PrP^{Sc} was achieved by repeating the amplification reactions, even if the brain homogenate had been diluted to a considerable extent.
Then, the amplification of PrP^{Sc} using a further diluted brain homogenate was examined.

### 1. Method

As with II of Test Example 2, a 10% brain homogenate derived from a scrapie-infected sheep was 10⁻² to 10⁻¹⁴-fold diluted and used.
PrP^{c} derived from an ICR mouse, which was a heterologous animal, was used as PrP^{c} as a source. A sample with no added source served as a control with no additive. Amplification was performed in duplicate.

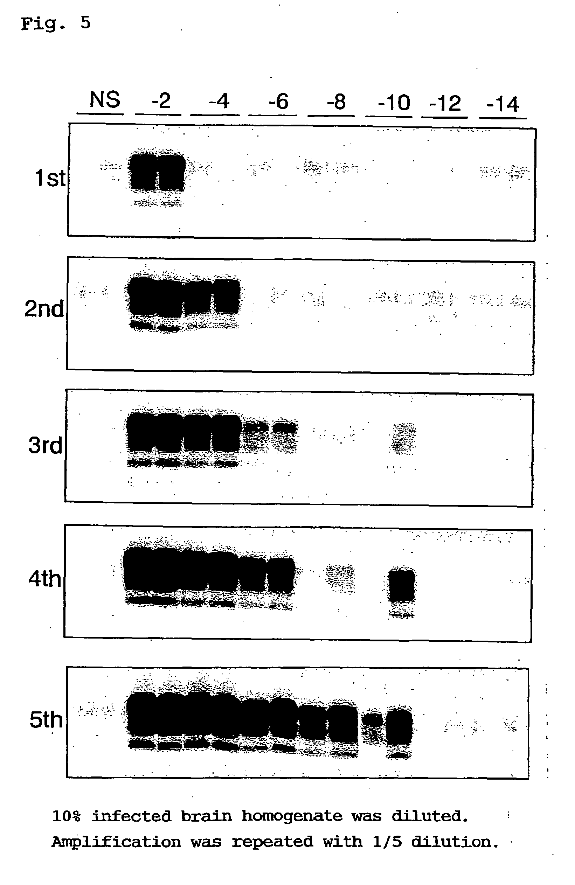

The results are shown in Fig. 5. As is understood from the results shown in Fig. 5, according to the method of the present invention, even if there is only a trace amount of PrP^{Sc}, amplification of PrP^{Sc} from a 10⁻¹⁰-fold diluted infected brain homogenate was achieved by performing 5 rounds of the amplification reaction repeatedly. The detection limit of hamster PrP^{Sc} when 6 to 7 rounds of amplification were performed is about 10⁻¹². The amount of PrP^{Sc} present in a brain homogenate derived from an infected sheep was estimated to be about one-hundredth to one-thousandth the amount of PrP^{Sc} included in a brain homogenate of an experimentally infected hamster by Western blot analysis. Considering this, a detection limit comparable to that of hamster PrP^{Sc} was achieved by the method of the present invention.

### Test Example 4: Amplification of PrP^{Sc} Derived from Sheep Scrapie (Amplification of Genotypically Different PrP^{Sc})

A sheep PrP gene is known to have mutations that cause various amino acid substitutions. Particularly, a combination of amino acids at 3 positions, amino acids 136 (A, V), 154 (R, H), and 171 (R, Q, H), is linked to sensitivity or resistance to scrapie. Then, the amplification of PrP^{Sc} derived from genotypically different scrapie-infected sheep was examined.

### 1. Method

Four types of sheep, that is, ARQ homozygous sheep, V/ARQ heterozygous sheep, AR/HQ heterozygous sheep, and AHQ homozygous sheep from the United Kingdom were used as genotypically different scrapie-infected sheep.
Brain homogenates derived from each type of sheep were 1/1, 1/5, 1/25, and 1/125 diluted with a normal mouse brain homogenate, and amplification was performed.

### 2. Result

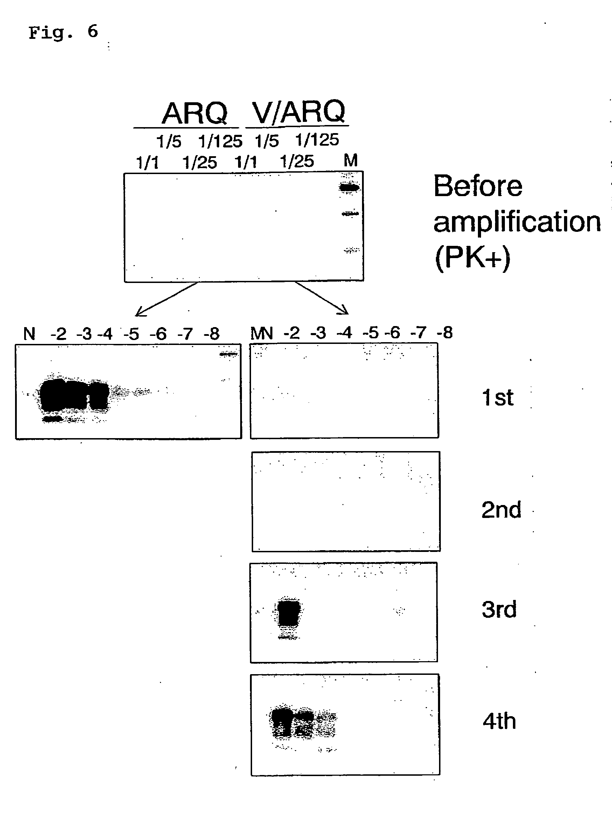

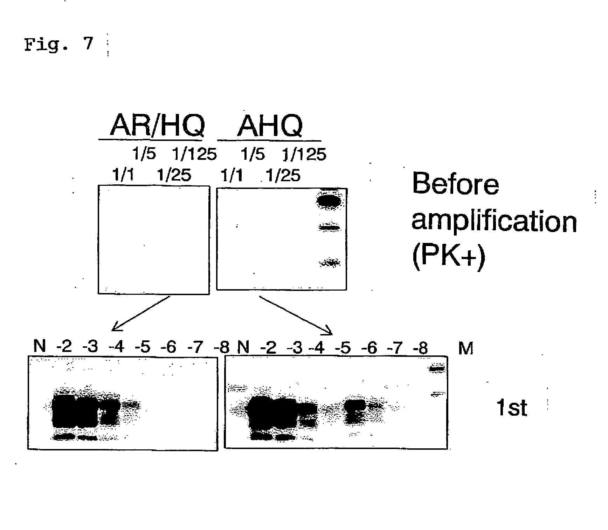

The results are shown in Figs. 6 and 7.
As is understood from the results shown in Figs. 6 and 7, it was found that the PrP^{Sc} examined, although it derived from 4 types of genotypically different sheep, could be amplified by the method of the present invention. The signal was observed after one round of amplification in sheep other than V/ARQ heterozygous sheep, while the signal was confirmed after 3 rounds of amplification in V/ARQ heterozygous sheep. Since the amount of PrP^{Sc} in the brain homogenates of each genotype was unknown, a possible reason is that the amount of PrP^{Sc} in the brain homogenate of V/ARQ type was less than that in the brain homogenates of other types or that the amplification efficiency of PrP^{Sc} derived from V/ARQ type was lower than that of other types.

### Test Example 5: Detection of PrP^{Sc} in Blood of Scrapie-Infected Sheep

The present invention enabled detection of a trace amount of PrP^{Sc} derived from sheep scrapie and more detailed analysis of kinetics of PrP^{Sc} in the body of an infected sheep than a conventional method.
Then, the amplification of PrP^{Sc} from blood was analyzed by using an experimentally scrapie-infected sheep.

### 1. Method

PrP^{Sc} derived from an experimentally scrapie-infected sheep was amplified using the PMCA method as with Test Example 1 described above. Blood was collected from two infected sheep presenting with the symptoms and centrifuged to obtain a leukocyte fraction. The leukocyte fraction was solubilized in a phosphate buffer which contained 2% sarcosyl and used as a PrP^{Sc} seed. PrP^{c} derived from an ICR mouse, which was a heterologous animal, was used as a PrP^{c} source. A sample with no added seed served as a control with no additive.

### 2. Result

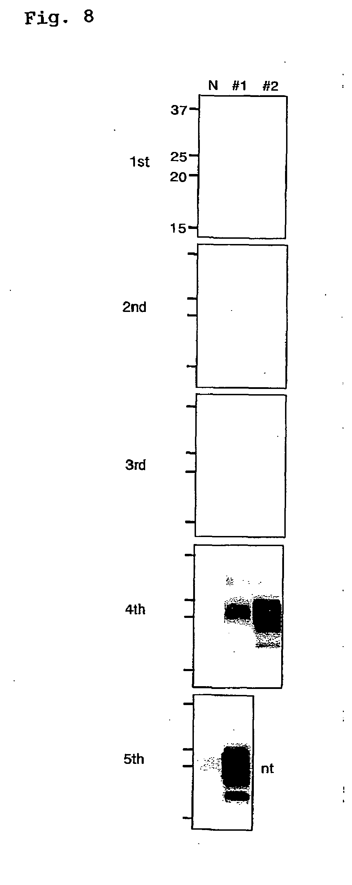

The results are shown in Fig. 8.
A clear PrP^{RES} signal was identified after 4 rounds of amplification in the case of a leukocyte fraction obtained from an infected sheep 2. A PrP^{RES} signal became clear after 5 rounds of amplification in the case of a leukocyte fraction obtained from an infected sheep 1.
In the figure, N indicates a control with no additive which does not contain a seed, and nt means that a test was not performed.
The results from this Test Example demonstrate that the method for amplifying PrP^{Sc} derived from sheep scrapie according to the present invention is also effective in a blood sample, and it is understood that the method is applicable to an antemortem diagnosis of scrapie.

### INDUSTRIAL APPLICABILITY

As described above, the method provided by the present invention enables an effective amplification of PrP^{Sc} derived from sheep scrapie and detection of a trace amount of PrP^{Sc}.
The sensitivity in detecting PrP^{Sc} derived from sheep scrapie by using the method of the present invention is remarkably efficient compared to existing detection methods such as the ELISA method. The method of the present invention is advantageous in that the sensitivity obtained after repeated amplification reactions will be higher than that in a bioassay, and thus it also has excellent practicality and rapidity.

Accordingly, the method of the present invention permits an antemortem diagnosis and an early diagnosis of sheep scrapie. Moreover, the method permits rapid examination of inactivation of abnormal prion protein derived from sheep scrapie, thereby contributing to establishing a method for inactivating abnormal prion protein. This method is also applicable to a safety evaluation method of raw materials for feed and fertilizer such as meat and bone meal, environmental monitoring of, for example, PrP^{Sc} in soil and the like. Furthermore, it is applicable to a communicable disease control measure for sheep scrapie for organism (animal) import, and furthermore, a preventive measure for imported mutton. Thus, this method is highly useful.

## Claims

1. A method for efficiently amplifying abnormal prion protein (PrP^{Sc}) derived from sheep scrapie, wherein the method is based on a PMCA (protein misfolding cyclic amplification) method in which normal prion protein (PrP^{c}) derived from mouse is used as a source and PrP^{Sc} is used as a seed, and PrP^{Sc} derived from sheep scrapie is amplified by mixing, incubating, and sonicating both the PrP^{c} and the PrP^{Sc} repeatedly.

2. The method for efficiently amplifying PrP^{Sc} derived from sheep scrapie according to claim 1, wherein the PrP^{c} used as a source derives from a brain homogenate of mouse containing PrP^{c}, and the PrP^{Sc} derived from sheep scrapie which is used as a seed derives from a body tissue containing PrP^{Sc} derived from a sheep scrapie-infected animal.

## Patentansprüche

1. Verfahren zur effizienten Amplifizierung von abnormalem Prion-Protein (PrP^{Sc}), abgeleitet von Schaf-Scrapie, wobei das Verfahren auf einem PMCA (Protein Misfolding Cyclic Amplification/Protein-Missfaltung zyklische Amplifizierung)-Verfahren basiert, in dem normales Prion-Protein (PrP^{c}), abgeleitet von der Maus, als eine Quelle verwendet wird, und PrP^{Sc} als Keim verwendet wird, und PrP^{Sc}, abgeleitet von Schaf-Scrapie, durch Mischen, Inkubieren und wiederholtes mit Ultraschall Behandeln, sowohl von dem PrP^{c} als auch dem PrP^{Sc}, amplifiziert wird.

2. Verfahren zur effizienten Amplifizierung von PrP^{Sc}, abgeleitet von Schaf-Scrapie gemäß Anspruch 1, wobei das PrP^{c}, das als eine Quelle verwendet wird, von einem Hirn-Homogenat einer Maus abgeleitet ist, das PrP^{c} enthält, und das PrP^{Sc}, abgeleitet von Schaf-Scrapie, das als ein Keim verwendet wird, von einem Körpergewebe abgeleitet ist, das PrP^{Sc}, abgeleitet von einem Schaf-Scrapie-infizierten Tier, enthält.

## Revendications

1. Un procédé pour l'amplification efficace d'une protéine prion anormale (PrP^{Sc}) provenant de la scrapie du mouton (la tremblante du mouton), le procédé étant basé sur une méthode de PMCA (amplification cyclique d'une protéine mal repliée), dans lequel la protéine prion normale (PrP^{c}) provenant de la souris est utilisée en tant que source et PrP^{Sc} est utilisée en tant que germe, et PrP^{Sc} provenant de la scrapie du mouton est amplifiée par le mélange, l'incubation et la sonication à la fois de la PrP^{c} et de la PrP^{Sc} à plusieurs reprises.

2. Le procédé pour l'amplification efficace de la PrP^{Sc} provenant de la scrapie du mouton selon la revendication 1, dans lequel la PrP^{c} qui est utilisée en tant que source, provient d'un homogénate de cerveau de souris contenant de la PrP^{c}, et la PrP^{Sc} provenant de la scrapie du mouton qui est utilisée en tant que germe provient d'un tissu corporel contenant de la PrP^{Sc} provenant d'un animal infecté par la scrapie de mouton.
